# EUROPEAN PATENT APPLICATION

(11) **EP 4 036 140 A1**
(43) Date of publication of application: **03.08.2022**
(21) Application number: 20870392.6
(22) Date of filing: 26.08.2020
(51) Int. Cl.: C08G 18/83, C08G 18/09, C08G 18/28, C08L 101/02

(54) **MODIFIED POLYCARBODIIMIDE COMPOUND HAVING HYDROPHILIC GROUP**

(30) Priority: 25.09.2019 JP 2019174214
(71) Applicant: Nisshinbo Chemical Inc., Tokyo 103-8650 (JP)
(72) Inventor: SASAKI, Takahiro, Chiba-city, Chiba 267-0056 (JP); FUKASE, Atsuko, Chiba-city, Chiba 267-0056 (JP)
(74) Representative: Mewburn Ellis LLP
(86) International application number: PCT/JP2020/032173
(87) International publication number: WO 2021/059835

(57) **Abstract**

A modified polycarbodiimide compound which is obtained by modifying at least some of carbodiimide groups in a polycarbodiimide derived from a diisocyanate compound, said polycarbodiimide having a terminal blocked with a hydrophilic compound, with an aromatic heterocyclic compound such as 3,5-dimethylpyrazole having endocyclic secondary amine nitrogen has a potential for a curing agent, and exhibits the activity of the carbodiimide groups by being unblocked at relatively low temperatures.

## Description

### TECHNICAL FIELD

This invention relates to a modified polycarbodiimide compound having a hydrophilic group.

### BACKGROUND ART

Epoxy compounds, oxazoline compounds and carbodiimide compounds are known to be curing agents which have a high reactivity with the carboxyl groups in carboxyl group-containing resins and exhibit an excellent curing performance.

Of these, carbodiimide compounds in particular have the advantage that they possess a high heat resistance and thus exhibit, in the cured product as well, an excellent heat resistance.

On the other hand, because of their high reactivity, carbodiimide compounds have a short pot life following mixture with a resin, making it necessary to carry out mixture just prior to use, in addition to which they undergo gelation when stored.

Techniques that block polycarbodiimides to increase their storage stability have been developed in order to resolve these problems. For example, Patent Document 1 discloses a method for grafting an unsaturated bond-containing reactive compound to some of the carbodiimide groups.

However, in this method, some carbodiimide groups remain present and so self-crosslinking reactions cannot be entirely eliminated.

Patent Document 2 discloses, as a method for enhancing storage stability when a carbodiimide compound and a resin have been mixed together, a one-pack epoxy resin composition containing a polyguanidine in which the carbodiimide groups on a polycarbodiimide compound are modified with a dialkylamine having linear or branched alkyl groups of four or more carbons. In this method, an excellent storage stability is exhibited for epoxy resins even when prepared as a one-pack composition, and curing can be effected at a relatively low temperature.

Yet, because the modifying amines on the modified polycarbodiimide compound of Patent Document 2 do not dissociate during curing, the polyguanidine must contribute to the curing reaction, making it difficult to effect the curing of carboxyl group-containing resins.

Patent Document 3 discloses a water-soluble or water-dispersible, amine-modified polycarbodiimide obtained by modifying with a secondary amine a polycarbodiimide which is end-capped with a hydrophilic compound.

However, because the secondary amine used in Patent Document 3 requires a relatively high temperature for dissociation and removal by volatilization, there is a concern that, under the low-temperature drying conditions commonly used in aqueous or water-dispersible systems, the curing reaction will not proceed and the desired performance cannot be manifested.

### PRIOR ART DOCUMENTS

### PATENT DOCUMENTS

Patent Document 1: JP-A H07-330849
Patent Document 2: JP-A 2000-136231
Patent Document 3: JP-A 2013-112755

### SUMMARY OF INVENTION

### TECHNICAL PROBLEM

The present invention was arrived at in light of the above circumstances. An object of the invention is to provide a hydrophilic group-containing modified polycarbodiimide compound which has latency as a curing agent and is deblocked at a relatively low temperature, allowing carbodiimide group activity to emerge.

### SOLUTION TO PROBLEM

The inventors have conducted intensive investigations in order to achieve the above object. As a result, they have discovered that by modifying the carbodiimide groups on a hydrophilic group-containing polycarbodiimide compound with an aromatic heterocyclic compound having an endocyclic secondary amine nitrogen so as to block the carbodiimide groups, deblocking and regeneration of the carbodiimide groups occurs at a relatively low temperature, enabling the polycarbodiimide compound to function as a curing agent for carboxyl group-containing resins and the like.

Accordingly, the invention provides:
1. A modified polycarbodiimide compound obtained by modifying, with an aromatic heterocyclic compound having an endocyclic secondary amine nitrogen, at least some portion of the carbodiimide groups on a polycarbodiimide which is derived from a diisocyanate compound and is end-capped with a hydrophilic compound;
2. The modified polycarbodiimide compound of 1 above, wherein the aromatic heterocyclic compound contains two or more endocyclic nitrogens;
3. The modified polycarbodiimide compound of 1 or 2 above, wherein the aromatic heterocyclic compound is one or more selected from the group consisting of pyrazole compounds and imidazole compounds;
4. The modified polycarbodiimide compound of 3 above, wherein the aromatic heterocyclic compound is one or more selected from the group consisting of 3,5-dimethylpyrazole, 2-methylimidazole and imidazole;
5. The modified polycarbodiimide compound of any of 1 to 4 above, wherein the diisocyanate compound is an aromatic diisocyanate compound;
6. The modified polycarbodiimide compound of 5 above, wherein the aromatic diisocyanate compound is one or more selected from the group consisting of 2,4-tolylene diisocyanate, 2,6-tolylene diisocyanate, 2,4'-diphenylmethane diisocyanate, 4,4'-diphenylmethane diisocyanate and o-tolidine diisocyanate;
7. A curing agent which includes a modified polycarbodiimide compound of any of 1 to 6 above;
8. A heat-curable resin composition which includes the curing agent of 7 above and a resin that has reactive groups selected from the group consisting of carboxyl, amino and hydroxyl groups;
9. The heat-curable resin composition of 8 above, wherein the content of the curing agent is from 0.5 to 1.5 equivalents per equivalent of reactive groups on the resin; and
10. The heat-curable resin composition of 8 or 9 above, wherein the reactive group-containing resin is one or more selected from the group consisting of polyurethane resins, polyamide resins, acrylic resins, vinyl acetate resins, polyolefin resins and polyimide resins.

### ADVANTAGEOUS EFFECTS OF INVENTION

Because carbodiimide groups on the modified polycarbodiimide compound of the invention are blocked by an aromatic heterocyclic compound having an endocyclic secondary amine nitrogen, the active hydrogens in the guanidine following modification have a decreased electron density, enabling dissociation to be effected at a low temperature and in a short time.

The modified polycarbodiimide of the invention possessing such a characteristic is suitable as a curing agent for resins having groups that react with carbodiimide groups, such as carboxyl group-containing resins. A mixture of a curing agent containing the modified polycarbodiimide of the invention with a resin having reactive groups such as carboxyl groups, after being dried without curing under very mild temperature conditions, can be cured under mild conditions.

Compositions obtained by mixing the modified polycarbodiimide compound of the invention with an aqueous or water-dispersible resin containing reactive groups such as carboxyl groups have a long pot life.

### DESCRIPTION OF EMBODIMENTS

The invention is described more fully below.

The modified polycarbodiimide compound of the invention is characterized by being obtained via modification, with an aromatic heterocyclic compound having an endocyclic secondary amine nitrogen, of at least some portion of the carbodiimide groups on a polycarbodiimide which is derived from a diisocyanate compound and is end-capped with a hydrophilic compound.

### (1) Diisocyanate Compound

The diisocyanate compound used as the starting material for the modified polycarbodiimide compound of the invention is not particularly limited; a diisocyanate compound selected from among various known diisocyanate compounds may be suitably used for this purpose.

Specific examples include aliphatic isocyanates such as hexamethylene diisocyanate, 1,4-tetramethylene diisocyanate, 1,5-diisocyanato-2-methylpentane and lysine diisocyanate; alicyclic diisocyanates such as isophorone diisocyanate, norbornane diisocyanate, hydrogenated tolylene diisocyanate, hydrogenated xylene diisocyanate, hydrogenated diphenylmethane diisocyanate and hydrogenated tetramethylxylene diisocyanate; and aromatic diisocyanates such as 2,4'-diphenylmethane diisocyanate, 4,4'-diphenylmethane diisocyanate, 4,4'-diphenyl ether diisocyanate, p-phenylene diisocyanate, m-phenylene diisocyanate, 3,3'-dimethoxy-4,4'-biphenyl diisocyanate, o-tolidene diisocyanate, naphthalene diisocyanate, 2,4-tolylene diisocyanate, 2,6-tolylene diisocyanate, 3,3'-dimethyl-4,4'-diphenyl ether diisocyanate and 3,3'-dimethyl-4,4'-diphenyl ether diisocyanate. These may be used singly, or two or more may be used in combination.

Of these, from the standpoint of obtaining a polycarbodiimide compound of excellent heat resistance, an aromatic diisocyanate is preferred; 2,4'-diphenylmethane diisocyanate, 4,4'-diphenylmethane diisocyanate, 2,4-tolylene diisocyanate, 2,6-tolylene diisocyanate and o-tolidene diisocyanate are more preferred.

### (2) Hydrophilic Compound

The hydrophilic compound used as the end-capping agent is not particularly limited, provided that it is a hydrophilic organic compound which has hydrophilicity and also has reactivity with a terminal isocyanate group on the diisocyanate compound or the polycarbodiimide compound.

Specific examples of the hydrophilic compound include, but are not limited to, polyalkylene oxides of general formula (a) below that are end-capped with an alkoxy group or a phenoxy group, dialkylamino alcohols of general formula (b) below, alkyl hydroxycarboxylates of general formula (c) below, dialkylaminoalkylamines of general formula (d) below and alkyl sulfonates of general formula (e) below.

R¹-O-(CH₂-CHR²-O)ₘ-H (a)

(R³)₂-N-CH₂-CHR⁴-OH (b)

R⁵-O-CO-CHR⁶-OH (c)

(R⁷)₂-N-R⁸-NH₂ (d)

HO-R⁹-SO₃M (e)

In formula (a), R¹ is an alkyl group of 1 to 4 carbon atoms or a phenyl group, R² is a hydrogen atom or an alkyl group of 1 to 4 carbon atoms, and m is an integer from 1 to 30. In formula (b), R³ is an alkyl group of 1 to 4 carbon atoms and R⁴ is a hydrogen atom or an alkyl group of 1 to 4 carbon atoms. In formula (c), R⁵ is an alkyl group of 1 to 3 carbon atoms and R⁶ is a hydrogen atom or an alkyl group of 1 to 3 carbon atoms. In formula (d), R⁷ is an alkyl group of 1 to 4 carbon atoms and R⁸ is a hydrogen atom or an alkyl group of 1 to 4 carbon atoms. In formula (e), R⁹ is an alkylene group of 1 to 10 carbon atoms and M is an alkali metal such as sodium or potassium.

Specific examples of alkyl groups of 1 to 4 carbon atoms include methyl, ethyl, n-propyl, isopropyl, n-butyl, s-butyl, isobutyl and t-butyl.

Specific examples of alkyl groups of 1 to 3 carbon atoms include methyl, ethyl, n-propyl and isopropyl groups.

Specific examples of alkylene groups of 1 to 10 carbon atoms include methylene, ethylene, propylene, trimethylene, tetramethylene, pentamethylene, hexamethylene, heptamethylene, octamethylene, nonamethylene and decamethylene groups.

The hydrophilic compounds of general formulas (a) to (e) may be used singly or two or more may be used in combination.

In cases where there is a need to impart a high hydrophilicity in order to dissolve or disperse the polycarbodiimide in an aqueous solvent, such as in cases where the polycarbodiimide has a high degree of polymerization or in cases where there is a high proportion of water in the aqueous solvent used when, as subsequently described, the polycarbodiimide serves as a resin crosslinking agent, of the hydrophilic compounds of general formulas (a) to (e) above, the hydrophilic compound is preferably a polyalkylene oxide of general formula (a) that is end-capped with an alkoxy group or a phenoxy group and has an excellent hydrophilicity.

Specific examples of the polyalkylene oxide of general formula (a) include polyethylene glycol monomethyl ether, polyethylene glycol monoethyl ether, polypropylene glycol monomethyl ether, polypropylene glycol monoethyl ether and polypropylene glycol monophenyl ether. Polyethylene glycol monomethyl ether is especially preferred.

In addition to the above hydrophilic compound, a compound which is capable of reacting with a terminal isocyanate group and which cannot by itself impart sufficient hydrophilicity to the polycarbodiimide compound may be concomitantly used as another end-capping agent in the polycarbodiimide compound of the invention.

The other end-capping agent is mixed with the above-described hydrophilic compound and used in a range where the polycarbodiimide exhibits a hydrophilicity that enables it to dissolve in an aqueous medium.

The other end-capping agent is not particularly limited, provided that it is a compound having reactivity with an isocyanate group at the end of the diisocyanate compound or the polycarbodiimide.

The other end-capping agent is exemplified by aliphatic compounds, aromatic compounds and alicyclic compounds having a functional group which reacts with an isocyanate groups. Specific examples include -OH group-containing methanol, ethanol, phenol, cyclohexanol and N-methylethanolamine; =NH group-containing diethylamine and dicyclohexylamine; -NH₂ group-containing butylamine and cyclohexylamine; -COOH group-containing propionic acid, benzoic acid and cyclohexanecarboxylic acid; -SH group-containing ethylmercaptan, allylmercaptan and thiophenol; and epoxy group-containing compounds.

The other end-capping agent may be used singly or two or more may be used in combination.

Alternatively, in order to cap the ends of the polycarbodiimide and control the degree of polymerization thereof, a monoisocyanate may be used as the end-capping agent.

Specific examples of the monoisocyanate include phenyl isocyanate, p-nitrophenyl isocyanate, p- and m-tolyl isocyanate, p-formylphenyl isocyanate and p-isopropylphenyl isocyanate. These may be used singly or two or more may be used in combination.

Of these, p-isopropylphenyl isocyanate is preferred.

The reaction of terminal isocyanate groups with the end-capping agent readily proceeds by mixing together the diisocyanate compound or polycarbodiimide with the end-capping agent at a normal temperature of about 25°C, although heating may be carried out if necessary.

It is preferable at this time to use the end-capping agents (the sum of the hydrophilic compound and other, optional, end-capping agents that are used) in a chemically equivalent amount with respect to the terminal isocyanate groups to be capped. The reaction is preferably carried out in an inert gas atmosphere.

### (3) Polycarbodiimide Compound

The diisocyanate compound-derived polycarbodiimide compound used in this invention has groups of general formula (1) below. 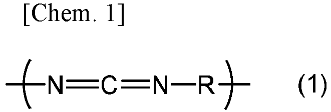 (wherein R represents a residual group obtained by removing an isocyanate (NCO) group from a diisocyanate compound).

The polycarbodiimide compound used in this invention may be a polycarbodiimide copolymer synthesized using at least one diisocyanate compound and having at least two carbodiimide groups on the molecule.

Such a copolymer can be obtained by copolymerizing a polycarbodiimide with, for example, a polyether polyol, a polyester polyol, a polycarbonate polyol or a polybutadiene diol.

The polycarbodiimide compound can be prepared by various methods that use a diisocyanate compound as the starting material. A typical method of preparation involves preparing an isocyanate-terminated polycarbodiimide by the decarboxylative condensation of a diisocyanate compound accompanied by carbon dioxide removal (see, for example, U.S. Pat. No. 2,941,956, JP-B S47-33279, J. Org. Chem. 28, 2069-2075 (1963), and Chemical Review 1981, Vol. 81, No. 4, pp 619-621).

A carbodiimidization catalyst is generally used in the decarboxylative condensation of a diisocyanate compound.

Specific examples of carbodiimidization catalysts include phospholene oxides such as 1-phenyl-2-phospholene-1-oxide, 3-methyl-1-phenyl-2-phospholene-1-oxide, 1-ethyl-2-phospholene-1-oxide, 3-methyl-2-phospholene-1-oxide, and 3-phospholene isomers of these. These may be used singly or two or more may be used in combination.

Of these, from the standpoint of reactivity, 3-methyl-1-phenyl-2-phospholene-1-oxide is preferred.

The amount of carbodiimidization catalyst is typically from 0.1 to 1.0 wt% with respect to the diisocyanate compound.

The above decarboxylative condensation reaction may be carried out in the absence of a solvent, although a solvent may be used.

Specific examples of solvents that may be used include alicyclic ethers such as tetrahydrofuran, 1,3-dioxane and dioxolane; aromatic hydrocarbons such as benzene, toluene, xylene and ethylbenzene; halogenated hydrocarbons such as chlorobenzene, dichlorobenzene, trichlorobenzene, perclene, trichloroethane and dichloroethane; ester compounds such as ethyl acetate and butyl acetate; and ketone compounds such as methyl ethyl ketone, methyl isobutyl ketone and cyclohexanone. These may be used singly or two or more may be used in combination.

Of these, cyclohexanone and tetrahydrofuran are preferred.

The reaction temperature, although not particularly limited, is preferably from 40 to 200°C, and more preferably from 50 to 130°C. When the reaction is carried out in a solvent, a reaction temperature of from 40°C to the boiling point of the solvent is preferred.

When the reaction is carried out in a solvent, the concentration of the diisocyanate compound is not particularly limited. However, in order to have the reaction proceed efficiently while suppressing gelation, the concentration is preferably from 5 to 55 wt%, and more preferably from 5 to 40 wt%.

The solids concentration within the reaction system, although not particularly limited, is preferably from 5 to 55 wt%, and more preferably from 20 to 50 wt%.

Particularly in cases where a catalyst is added and carbodiimidization is carried out after the diisocyanate compound and the end-capping agent have been reacted and the ends thereby capped, the reaction temperature is preferably from 40 to 180°C, and more preferably from 50 to 100°C.

In this case, the concentration of the diisocyanate compound in the solvent is preferably from 5 to 55 wt%, and more preferably from 20 to 50 wt%.

The degree of polymerization of the polycarbodiimide used in this invention is not particularly limited. However, to increase the solubility or dispersibility in water and also to efficiently suppress gelation within an aqueous medium, the degree of polymerization is preferably from 2 to 15, and more preferably from 3 to 12.

When preparing the polycarbodiimide compound of the invention, a polyol having two or more hydroxyl groups on the molecule may be concomitantly used. Introducing a polyol component relaxes the strong cohesive forces of the modified carbodiimide compound of the invention, enabling compatibility with aqueous solvents to be improved.

Such polyols are exemplified by polyester polyols, polyether polyols, polycarbonate polyols, castor oil polyols and long-chain aliphatic diols.

The castor oil polyol may be one derived from castor oil. Specific examples include URIC H-30, URIC H-62 and URIC Y-403 (from Ito Oil Chemicals Co., Ltd.); and HS 2G-120, HS SG-160R, HS 2G-270B, HS 2B-5500 and HS KA-001 (from Hokoku Corporation).

Examples of polyether polyols include polyethylene glycol, polypropylene glycol and polytetramethylene glycol. Specific examples include Sannix PP-400, Sannix PP-1000 and Sannix PP-2000 (from Sanyo Chemical, Ltd.); and Uniol PB-500 and Uniol PB-700 (from NOF Corporation).

Of these, from the standpoint of compatibility, a castor oil polyol is preferred, and a castor oil polyol having two functional groups is more preferred.

### (4) Aromatic Heterocyclic Compound Having Endocyclic Secondary Amine Nitrogen

In this invention, the aromatic heterocyclic compound used to modify the above polycarbodiimide compound is an aromatic heterocyclic compound having an endocyclic secondary amine nitrogen. As used herein, "aromatic heterocyclic compound having an endocyclic secondary amine nitrogen" refers to an aromatic heterocyclic compound having an amine on the heterocycle.

The aromatic heterocyclic compound is not particularly limited, provided that it has an endocyclic secondary amine nitrogen. Specific examples include pyrrole compounds, pyrazole compounds, imidazole compounds and triazole compounds. From the standpoint of further lowering the temperature at the onset of dissociation from the modified polycarbodiimide compound, an aromatic heterocyclic compound in which the number of endocyclic nitrogens is two or more is preferred; pyrazole compounds and imidazole compounds are more preferred.

Specific examples of pyrazole compounds include pyrazole, 3-methylpyrazole, 4-methylpyrazole and 3,5-dimethylpyrazole.

Specific examples of imidazole compounds include imidazole, 2-methylimidazole, 2-ethyl-4-methyl-imidazole, 2-phenylimidazole and 2-phenyl-4-methylimidazole.

Of these, 3,5-dimethylpyrazole, 2-methylimidazole and imidazole are preferred.

### (5) Modification of Polycarbodiimide Compound

The modified polycarbodiimide compound of the invention is obtained by modifying a polycarbodiimide compound with an aromatic heterocyclic compound having an endocyclic secondary amine nitrogen.

This modification may be carried out by mixing an aromatic heterocyclic compound having an endocyclic secondary amine nitrogen and a polycarbodiimide compound to a given number of equivalents of the aromatic heterocyclic compound with respect to the carbodiimide groups and effecting the reaction.

This reaction may be carried out in the absence of solvents, although it is preferable to use an aqueous solvent.

In cases where an aqueous solvent is used, the reaction may be effected by mixing the polycarbodiimide compound with the aqueous solvent, and adding thereto the aromatic heterocyclic compound having an endocyclic secondary amine nitrogen to a given number of equivalents with respect to the carbodiimide groups.

The aromatic heterocyclic compound having an endocyclic secondary amine nitrogen is used in an amount which is not particularly limited, provided that the performance desired of the curing agent can be exhibited, although use in an amount of 1 to 2 equivalents per equivalent of carbodiimide groups is preferred. To reduce the amount of unreacted aromatic heterocyclic compound and facilitate amine removal at the time of heat treatment, use in an amount of from 1 to 1.5 equivalents is more preferred.

The reaction temperature is not particularly limited. However, to have the reaction proceed efficiently and also to suppress side reactions, the temperature is preferably room temperature (about 25°C) or from 40 to 120°C.

The reaction time fluctuates with the reaction temperature and therefore cannot be strictly specified, although the reaction time is typically from about 0.1 hour to about 2 hours.

The reaction is preferably carried out under stirring.

The aqueous solvent used in the amine modification reaction may be water alone, a hydratable liquid compound alone, or a mixed solvent of water with a hydratable liquid compound.

Specific examples of hydratable liquid compounds include polyalkylene glycol monoalkyl ethers such as ethylene glycol monomethyl ether, ethylene glycol monoethyl ether, ethylene glycol monobutyl ether, diethylene glycol monoethyl ether, diethylene glycol monobutyl ether, propylene glycol monomethyl ether and dipropylene glycol monomethyl ether; polyalkylene glycol dialkyl ethers such as diethylene glycol dimethyl ether, triethylene glycol dimethyl ether and dipropylene glycol dimethyl ether; polyalkylene glycol monoalkyl ether acetates such as ethylene glycol monomethyl ether acetate, ethylene glycol monoethyl ether acetate, ethylene glycol monobutyl ether acetate, diethylene glycol monoethyl ether acetate, diethylene glycol monobutyl ether acetate, propylene glycol monomethyl ether acetate and dipropylene glycol monomethyl ether acetate; polyalkylene glycol diacetates such as ethylene glycol diacetate and propylene glycol diacetate; polyalkylene glycol monophenyl ethers such as ethylene glycol monophenyl ether and propylene glycol monophenyl ether; monoalcohols such as propanol, butanol, hexanol and octanol; N-substituted amides such as N-methyl-2-pyrrolidone (NMP) and N-ethyl-2-pyrrolidone (NEP); and 2,2,4-trimethyl-1,3-pentanediol monoisobutylate. These may be used singly or two or more may be used in combination.

### (6) Curing Agent and Heat-Curable Resin Composition

The above-described modified polycarbodiimide compound of the invention can be suitably used as a resin curing agent or curing accelerator. Specifically, a curing agent containing the modified polycarbodiimide of the invention functions as a curing agent for resins having reactive groups that undergo crosslinking reactions with carbodiimide groups.

Specific examples of such resins include carboxyl group-containing resins having carboxyl groups on the molecule, amino group-containing resins having amino groups on the molecule, and hydroxyl group-containing resins having hydroxyl groups on the molecule.

In terms of the ease of carrying out crosslinking reactions with carbodiimide groups, the reactive group-containing resin is preferably a polyurethane resin, polyamide resin, acrylic resin, vinyl acetate resin, polyolefin resin or polyimide resin having reactive groups.

The heat-curable resin composition of the invention includes the above-described resin and the curing agent of the invention.

In the modified polycarbodiimide compound of the invention, because the carbodiimide groups are blocked by an aromatic heterocyclic compound having an endocyclic secondary amine nitrogen, the active hydrogens on the guanidine following modification have a decreased electron density, enabling dissociation to be effected at a low temperature and in a short time.

The heat-curable resin composition of the invention containing such a curing agent, after being dried without curing under very mild temperature conditions, can be cured under mild conditions and also has a long pot life. Hence, the extended shelf life and handleability are excellent.

In the heat-curable resin composition of the invention, the content of curing agent is not particularly limited so long as the desired durability is exhibited. To achieve a suitable resin curability, the amount of curing agent is preferably from 0.5 to 1.5 equivalents, and more preferably from 0.8 to 1.2 equivalents, per equivalent of reactive groups in the base resin. Little additional change in the advantageous effects is achieved by setting the content of the curing agent to more than 1.5 equivalents.

Depending on the intended use, where necessary, the heat-curable resin composition of the invention may suitably include various added ingredients, such as pigments, fillers, leveling agents, surfactants, dispersants, plasticizers, ultraviolet absorbers and antioxidants.

A film can be produced by applying the heat-curable resin composition of the invention onto a given substrate to form an applied layer, and curing the applied layer.

Known methods may be suitably used here as the method of application. Examples of such methods include brush coating, padding, spray coating, hot spray coating, airless spray coating, roller coating, curtain flow coating, flow coating, dip coating and knife edge coating.

After forming the applied layer, heat treatment may be carried out to accelerate the crosslinking reactions. The heating method is not particularly limited. For example, an electric heating oven, an infrared heating oven or a high-frequency heating oven may be used.

### EXAMPLES

Examples and Comparative Examples are given below to more fully illustrate the invention, although the invention is not limited by these Examples.

### [1] Synthesis of Modified Polycarbodiimide Compounds

### [Example 1-1]

A polycarbodiimide compound was prepared by placing 100 parts by weight of tolylene diisocyanate (abbreviated below as "TDI"; Cosmonate T-80, from Mitsui Chemicals, Inc.), 105 parts by weight of polyethylene glycol monomethyl ether (molecular weight, 550; Braunon MP-550, from Aoki Oil Industrial Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 3 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 495 parts by weight of water, 43.2 parts by weight of 2-methylimidazole (abbreviated below as "2MZ"; Curezol 2MZ-H, from Shikoku Chemicals Corporation) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the 2MZ-modified polycarbodiimide compound P1 (n = 5; carbodiimide equivalent weight, 385 g/mol) was obtained.

### [Example 1-2]

A polycarbodiimide compound was prepared by placing 100 parts by weight of diphenylmethane diisocyanate (abbreviated below as "MDI" (here and below, a mixture of 54% 2,4'-MDI and 46% 4,4'-MDI); Millionate NM, from Tosoh Corporation), 64.0 parts by weight of polyethylene glycol monomethyl ether (molecular weight, 550; Braunon MP-550, from Aoki Oil Industrial Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 409 parts by weight of water, 28.9 parts by weight of 2MZ (Curezol 2MZ-H, from Shikoku Chemicals Corporation) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the 2MZ-modified polycarbodiimide compound P2 (n = 4; carbodiimide equivalent weight, 469 g/mol) was obtained.

### [Example 1-3]

A polycarbodiimide compound was prepared by placing 100 parts by weight of TDI, 79.0 parts by weight of polyethylene glycol monomethyl ether (molecular weight, 400; Braunon MP-400, from Aoki Oil Industrial Co., Ltd.), 22.7 parts by weight of polypropylene glycol (molecular weight, 400; Sannix PP-400, from Sanyo Chemical, Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 513 parts by weight of water, 38.9 parts by weight of 2MZ (Curezol 2MZ-H, from Shikoku Chemicals Corporation) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the 2MZ-modified polycarbodiimide compound P3 (n = 6; carbodiimide equivalent weight, 438 g/mol) was obtained.

### [Example 1-4]

A polycarbodiimide compound was prepared by placing 100 parts by weight of MDI, 62.9 parts by weight of polyethylene glycol monomethyl ether (molecular weight, 550; Braunon MP-550, from Aoki Oil Industrial Co., Ltd.), 25.1 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 530 parts by weight of water, 25.8 parts by weight of 2MZ (Curezol 2MZ-H, from Shikoku Chemicals Corporation) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the 2MZ-modified polycarbodiimide compound P4 (n = 5; carbodiimide equivalent weight, 666 g/mol) was obtained.

### [Example 1-5]

A polycarbodiimide compound was prepared by placing 100 parts by weight of TDI, 52.7 parts by weight of polyethylene glycol monomethyl ether (molecular weight, 550; Braunon MP-550, from Aoki Oil Industrial Co., Ltd.), 17.7 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 440 parts by weight of propylene glycol monomethyl ether, 43.2 parts by weight of 2MZ (Curezol 2MZ-H, from Shikoku Chemicals Corporation) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the 2MZ-modified polycarbodiimide compound P5 (n = 10; carbodiimide equivalent weight, 345 g/mol) was obtained.

### [Example 1-6]

A polycarbodiimide compound was prepared by placing 100 parts by weight of TDI, 52.7 parts by weight of polyethylene glycol monomethyl ether (molecular weight, 550; Braunon MP-550, from Aoki Oil Industrial Co., Ltd.), 17.7 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 440 parts by weight of a mixed solvent of propylene glycol monomethyl ether and water (1/1, w/w), 43.2 parts by weight of 2MZ (Curezol 2MZ-H, from Shikoku Chemicals Corporation) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the 2MZ-modified polycarbodiimide compound P6 (n = 10; carbodiimide equivalent weight, 345 g/mol) was obtained.

### [Example 1-7]

A polycarbodiimide compound was prepared by placing 100 parts by weight of TDI, 65.7 parts by weight of polyethylene glycol monomethyl ether (molecular weight, 550; Braunon MP-550, from Aoki Oil Industrial Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 3 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 369 parts by weight of water, 52.0 parts by weight of 3,5-dimethylpyrazole (abbreviated below as "DMP"; from Japan Finechem Co., Inc.) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DMP-modified polycarbodiimide compound P7 (n = 6; carbodiimide equivalent weight, 292 g/mol) was obtained.

### [Example 1-8]

A polycarbodiimide compound was prepared by placing 100 parts by weight of TDI, 63.2 parts by weight of polyethylene glycol monomethyl ether (molecular weight, 550; Braunon MP-550, from Aoki Oil Industrial Co., Ltd.), 25.4 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 499 parts by weight of water, 36.4 parts by weight of DMP (Japan Finechem Co., Inc.) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DMP-modified polycarbodiimide compound P8 (n = 8; carbodiimide equivalent weight, 399 g/mol) was obtained.

### [Example 1-9]

A polycarbodiimide compound was prepared by placing 100 parts by weight of MDI, 73.3 parts by weight of polyethylene glycol monomethyl ether (molecular weight, 550; Braunon MP-550, from Aoki Oil Industrial Co., Ltd.), 19.6 parts by weight of polypropylene glycol (molecular weight, 400; Sannix PP-400, from Sanyo Chemical, Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 522 parts by weight of water, 28.2 parts by weight of DMP (Japan Finechem Co., Inc.) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DMP-modified polycarbodiimide compound P9 (n = 4; carbodiimide equivalent weight, 706 g/mol) was obtained.

### [Example 1-10]

A polycarbodiimide compound was prepared by placing 100 parts by weight of TDI, 52.7 parts by weight of polyethylene glycol monomethyl ether (molecular weight, 550; Braunon MP-550, from Aoki Oil Industrial Co., Ltd.), 17.7 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 432 parts by weight of ethylene diglycol acetate, 50.6 parts by weight of DMP (Japan Finechem Co., Inc.) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DMP-modified polycarbodiimide compound P10 (n = 10; carbodiimide equivalent weight, 345 g/mol) was obtained.

### [Example 1-11]

A polycarbodiimide compound was prepared by placing 100 parts by weight of TDI, 52.7 parts by weight of polyethylene glycol monomethyl ether (molecular weight, 550; Braunon MP-550, from Aoki Oil Industrial Co., Ltd.), 17.7 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 432 parts by weight of a mixed solvent of ethylene diglycol acetate and water (1/1, w/w), 50.6 parts by weight of DMP (Japan Finechem Co., Inc.) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DMP-modified polycarbodiimide compound P11 (n = 10; carbodiimide equivalent weight, 345 g/mol) was obtained.

### [Example 1-12]

A polycarbodiimide compound was prepared by placing 100 parts by weight of TDI, 105 parts by weight of polyethylene glycol monomethyl ether (molecular weight, 550; Braunon MP-550, from Aoki Oil Industrial Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 3 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 503 parts by weight of water, 35.8 parts by weight of imidazole (abbreviated below as "IMZ"; Curezol SIZ, from Shikoku Chemicals Corporation) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the IMZ-modified polycarbodiimide compound P12 (n = 5; carbodiimide equivalent weight, 385 g/mol) was obtained.

### [Comparative Example 1-1]

A polycarbodiimide compound was prepared by placing 100 parts by weight of TDI, 105 parts by weight of polyethylene glycol monomethyl ether (molecular weight, 550; Braunon MP-550, from Aoki Oil Industrial Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 3 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 485 parts by weight of water, 53.2 parts by weight of diisopropylamine (abbreviated below as DIPA) was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DIPA-modified polycarbodiimide compound P13 (n = 5; carbodiimide equivalent weight, 385 g/mol) was obtained.

### [Comparative Example 1-2]

A polycarbodiimide compound was prepared by placing 100 parts by weight of MDI, 64.0 parts by weight of polyethylene glycol monomethyl ether (molecular weight, 550; Braunon MP-550, from Aoki Oil Industrial Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 403 parts by weight of water, 35.6 parts by weight of DIPA was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DIPA-modified polycarbodiimide compound P14 (n = 4; carbodiimide equivalent weight, 469 g/mol) was obtained.

### [Comparative Example 1-3]

A polycarbodiimide compound was prepared by placing 100 parts by weight of TDI, 79.0 parts by weight of polyethylene glycol monomethyl ether (molecular weight, 400; Braunon MP-400, from Aoki Oil Industrial Co., Ltd.), 22.7 parts by weight of polypropylene glycol (molecular weight, 400; Sannix PP-400, from Sanyo Chemical, Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 504 parts by weight of water, 47.9 parts by weight of DIPA was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DIPA-modified polycarbodiimide compound P15 (n = 6; carbodiimide equivalent weight, 438 g/mol) was obtained.

### [Comparative Example 1-4]

A polycarbodiimide compound was prepared by placing 100 parts by weight of TDI, 52.7 parts by weight of polyethylene glycol monomethyl ether (molecular weight, 550; Braunon MP-550, from Aoki Oil Industrial Co., Ltd.), 17.7 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 432 parts by weight of a mixed solvent of ethyl diglycol acetate and water (1/1, w/w), 50.6 parts by weight of DIPA was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DIPA-modified polycarbodiimide compound P16 (n = 10; carbodiimide equivalent weight, 345 g/mol) was obtained.

### [Comparative Example 1-5]

A polycarbodiimide compound was prepared by placing 100 parts by weight of TDI, 52.7 parts by weight of polyethylene glycol monomethyl ether (molecular weight, 550; Braunon MP-550, from Aoki Oil Industrial Co., Ltd.), 17.7 parts by weight of castor oil polyol (molecular weight, 700; URIC Y-403, from Ito Oil Chemicals Co., Ltd.) and 1.0 part by weight of a carbodiimidization catalyst (3-methyl-1-phenyl-2-phospholene-1-oxide) in a reaction vessel fitted with a reflux condenser and a stirrer, stirring the vessel contents for 4 hours at 80°C under a stream of nitrogen, and confirming in infrared (IR) absorption spectroscopy the substantial disappearance of an absorption peak attributable to isocyanate groups at a wavelength of about 2270 cm⁻¹.

The resulting polycarbodiimide compound was mixed with 432 parts by weight of ethyl diglycol acetate, 50.6 parts by weight of DIPA was added thereto, and the mixture was cooled to room temperature and then stirred for 5 hours. Infrared (IR) absorption spectroscopy confirmed the formation of an absorption peak attributable to guanidine groups at a wavelength of about 1740 cm⁻¹ and the substantial disappearance of an absorption peak attributable to carbodiimide groups at a wavelength of about 2150 cm⁻¹, indicating that the DIPA-modified polycarbodiimide compound P17 (n = 10; carbodiimide equivalent weight, 345 g/mol) was obtained.

### [Ease of Amine Dissociation]

The modified polycarbodiimide compounds obtained in each of the above Examples and Comparative Examples were heated at 120°C for 10 minutes and the absorption peak attributable to carbodiimide groups at a wavelength of about 2150 cm⁻¹ in infrared absorption (IR) spectroscopy was confirmed. The ratio of the carbodiimide group peak that formed with amine dissociation after heating was calculated relative to 100% for the peak attributable to carbodiimide groups on the polycarbodiimide compound prior to modification. The results are shown in Table 1. When the ratio of the peak that formed was 80% or more, the ease of dissociation was rated as "○"; when the ratio was less than 80%, the ease of dissociation was rated as "×."

**[Table 1]**

| | Ease of Amine Dissociation |
|---|---|
| Example 1-1 | ○ |
| Example 1-2 | ○ |
| Example 1-3 | ○ |
| Example 1-4 | ○ |
| Example 1-5 | ○ |
| Example 1-6 | ○ |
| Example 1-7 | ○ |
| Example 1-8 | ○ |
| Example 1-9 | ○ |
| Example 1-10 | ○ |
| Example 1-11 | ○ |
| Example 1-12 | ○ |
| Comparative Example 1-1 | × |
| Comparative Example 1-2 | × |
| Comparative Example 1-3 | × |
| Comparative Example 1-4 | × |
| Comparative Example 1-5 | × |

As shown in Table 1, compared with the modified polycarbodiimide compounds in the Comparative Examples, the ease of amine dissociation in the modified polycarbodiimide compounds obtained in Examples 1-1 to 1-12 was excellent.

### [2] Production of Heat-Curable Urethane Resin Compositions and Curing Agents

### [Examples 2-1 to 2-12, Comparative Examples 2-1 to 2-5]

Heat-curable urethane resin compositions were prepared by mixing the modified polycarbodiimide compounds obtained in Examples 1-1 to 1-12 and Comparative Examples 1-1 to 1-5 with a carboxyl group-containing aqueous polyurethane resin (Suncure 777, from The Lubrizol Corporation; solids content, 35 wt%) in a carboxyl group to carbodiimide group equivalent ratio of 1:1.

### (1) Pot Life Measurement

The state of each of the heat-curable urethane resin compositions prepared in Examples 2-1 to 2-12 and Comparative Examples 2-1 to 2-5 after being left to stand at 50°C for one week was observed. The results are shown in Table 2. Here and below, compositions that maintained a liquid state were rated as "○"; compositions that solidified were rated as "×".

### (2) Rubbing Test

Each of the heat-curable urethane resin compositions prepared in Examples 2-1 to 2-12 and Comparative Examples 2-1 to 2-5 was cast onto an aluminum panel and dried at 70°C for 5 minutes, forming a 20 µm thick coat. The coat was then heated at 150°C for 10 minutes and cured, producing a film.

Using ethanol as the solvent, double rubbing of the resulting film was performed with an ER-1B friction tester (Suga Test Instruments Co., Ltd.) under a load of 900 g/cm². The results are shown in Table 2. Evaluations were carried out using the following whitening scores (the same applies below).
5: No change
4: Faint whitening
3: Some whitening
2: Complete whitening
1: Some decomposition
0: Decomposition

**[Table 2]**

| | Aqueous resin composition | | | Evaluation | |
|---|---|---|---|---|---|
| | Polyurethane resin | Carbodiimide compound | | Rubbing test | Pot life test |
| | pbw | Type | pbw | 150°C, 10 min | 50°C, 1 week |
| Example 2-1 | 100 | P1 | 8 | 5 | ○ |
| Example 2-2 | 100 | P2 | 10 | 5 | ○ |
| Example 2-3 | 100 | P3 | 9 | 5 | ○ |
| Example 2-4 | 100 | P4 | 14 | 5 | ○ |
| Example 2-5 | 100 | P5 | 7 | 5 | ○ |
| Example 2-6 | 100 | P6 | 7 | 5 | ○ |
| Example 2-7 | 100 | P7 | 6 | 5 | ○ |
| Example 2-8 | 100 | P8 | 8 | 5 | ○ |
| Example 2-9 | 100 | P9 | 15 | 5 | ○ |
| Example 2-10 | 100 | P10 | 7 | 5 | ○ |
| Example 2-11 | 100 | P11 | 7 | 5 | ○ |
| Example 2-12 | 100 | P12 | 8 | 5 | ○ |
| Comparative Example 2-1 | 100 | P13 | 8 | 3 | × |
| Comparative Example 2-2 | 100 | P14 | 10 | 3 | × |
| Comparative Example 2-3 | 100 | P15 | 9 | 3 | × |
| Comparative Example 2-4 | 100 | P16 | 7 | 3 | × |
| Comparative Example 2-5 | 100 | P17 | 7 | 3 | × |

### [3] Production of Heat-Curable Acrylic Resin Compositions and Cured Products

### [Examples 3-1 to 3-12, Comparative Examples 3-1 to 3-5]

Heat-curable acrylic resin compositions were prepared by mixing the modified polycarbodiimide compounds obtained in Examples 1-1 to 1-12 and Comparative Examples 1-1 to 1-5 with an aqueous acrylic resin (PRIMAL AC-261P, from Dow Inc.; solids content, 50 wt%) in a carboxyl group to carbodiimide group equivalent ratio of 1:1.

### (1) Pot Life Measurement

The state of each of the heat-curable acrylic resin compositions prepared in Examples 3-1 to 3-12 and Comparative Examples 3-1 to 3-5 after being left to stand at 50°C for one week was observed. The results are shown in Table 3.

### (2) Rubbing Test

Each of the heat-curable acrylic resin compositions prepared in Examples 3-1 to 3-12 and Comparative Examples 3-1 to 3-5 was cast onto an aluminum panel and dried at 70°C for 5 minutes, forming a 20 µm thick coat. The coat was then heated at 150°C for 30 minutes and cured, producing a film.

Using ethanol as the solvent, double rubbing of the resulting film was performed with an ER-1B friction tester (Suga Test Instruments Co., Ltd.) under a load of 900 g/cm². The results are shown in Table 3.

**[Table 3]**

| | Aqueous resin composition | | | Evaluation | |
|---|---|---|---|---|---|
| | Acrylic resin | Carbodiimide compound | | Rubbing test | Pot life test |
| | pbw | Type | pbw | 150°C, 30 min | 50°C, 1 week |
| Example 3-1 | 100 | P1 | 8 | 3 | ○ |
| Example 3-2 | 100 | P2 | 10 | 3 | ○ |
| Example 3-3 | 100 | P3 | 9 | 3 | ○ |
| Example 3-4 | 100 | P4 | 14 | 3 | ○ |
| Example 3-5 | 100 | P5 | 7 | 3 | ○ |
| Example 3-6 | 100 | P6 | 7 | 3 | ○ |
| Example 3-7 | 100 | P7 | 6 | 3 | ○ |
| Example 3-8 | 100 | P8 | 8 | 3 | ○ |
| Example 3-9 | 100 | P9 | 15 | 3 | ○ |
| Example 3-10 | 100 | P10 | 7 | 3 | ○ |
| Example 3-11 | 100 | P11 | 7 | 3 | ○ |
| Example 3-12 | 100 | P12 | 8 | 3 | ○ |
| Comparative Example 3-1 | 100 | P13 | 8 | 2 | × |
| Comparative Example 3-2 | 100 | P14 | 10 | 2 | × |
| Comparative Example 3-3 | 100 | P15 | 9 | 2 | × |
| Comparative Example 3-4 | 100 | P16 | 7 | 2 | × |
| Comparative Example 3-5 | 100 | P17 | 7 | 2 | × |

As is apparent from the results in Tables 2 and 3, the heat-curable resin compositions prepared in the Examples of the invention had good pot lives and were fully cured under curing conditions of 30 minutes at 150°C.

That is, by modifying carbodiimide groups with a given amine-containing aromatic heterocyclic compound, gelation and solidification following mixture can be efficiently prevented. As a result, the pot life can be improved and the amine used for modification can be dissociated at a low temperature and in a short time, enabling a cured film to be obtained by rapid curing under mild conditions of 30 minutes at 150°C.

## Claims

1. A modified polycarbodiimide compound obtained by modifying, with an aromatic heterocyclic compound having an endocyclic secondary amine nitrogen, at least some portion of the carbodiimide groups on a polycarbodiimide which is derived from a diisocyanate compound and is end-capped with a hydrophilic compound.

2. The modified polycarbodiimide compound of claim 1, wherein the aromatic heterocyclic compound contains two or more endocyclic nitrogens.

3. The modified polycarbodiimide compound of claim 1 or 2, wherein the aromatic heterocyclic compound is one or more selected from the group consisting of pyrazole compounds and imidazole compounds.

4. The modified polycarbodiimide compound of claim 3, wherein the aromatic heterocyclic compound is one or more selected from the group consisting of 3,5-dimethylpyrazole, 2-methylimidazole and imidazole.

5. The modified polycarbodiimide compound of any one of claims 1 to 4, wherein the diisocyanate compound is an aromatic diisocyanate compound.

6. The modified polycarbodiimide compound of claim 5, wherein the aromatic diisocyanate compound is one or more selected from the group consisting of 2,4-tolylene diisocyanate, 2,6-tolylene diisocyanate, 2,4'-diphenylmethane diisocyanate, 4,4'-diphenylmethane diisocyanate and o-tolidine diisocyanate.

7. A curing agent comprising a modified polycarbodiimide compound of any one of claims 1 to 6.

8. A heat-curable resin composition comprising the curing agent of claim 7 and a resin that has reactive groups selected from the group consisting of carboxyl, amino and hydroxyl groups.

9. The heat-curable resin composition of claim 8, wherein the content of the curing agent is from 0.5 to 1.5 equivalents per equivalent of reactive groups on the resin.

10. The heat-curable resin composition of claim 8 or 9, wherein the reactive group-containing resin is one or more selected from the group consisting of polyurethane resins, polyamide resins, acrylic resins, vinyl acetate resins, polyolefin resins and polyimide resins.
